(19) Europäisches Patentamt
European Patent Office
Office européen des brevets

(11) Veröffentlichungsnummer: **0 013 957**
**B1**

(12) # EUROPÄISCHE PATENTSCHRIFT

(45) Veröffentlichungstag der Patentschrift: **17.03.82**

(51) Int. Cl.³: **C 07 C 68/00**, C 08 G 63/62

(21) Anmeldenummer: **80100265.0**

(22) Anmeldetag: **21.01.80**

(54) **Verfahren zur Herstellung von Kohlensäureestern und seine Anwendung zur Herstellung von Polycarbonaten.**

(30) Priorität: **30.01.79 DE 2903506**

(43) Veröffentlichungstag der Anmeldung: **06.08.80 (Patentblatt 80/16)**

(45) Bekanntmachung des Hinweises auf die Patenterteilung: **17.03.82 Patentblatt 82/11**

(84) Benannte Vertragsstaaten: **DE FR GB IT NL**

(56) Entgegenhaltungen: **US - A - 2 834 799**

(73) Patentinhaber: **BAYER AG**
**Zentralbereich Patente, Marken und Lizenzen**
**D-5090 Leverkusen 1, Bayerwerk (DE)**

(72) Erfinder: **Heitz, Walter, Prof. Dr.**
**Am Schmidtborn 5**
**D-3575 Kirchhain 1 (DE)**
Erfinder: **Ball, Peter, Dr.**
**Am Burger Wald 71**
**D-8267 Neu Ötting (DE)**

**EP 0 013 957 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung von Kohlensäureestern und seine Anwendung zur Herstellung von Polycarbonaten

Gegenstand der vorliegenden Erfindung ist ein Verfahren zur Herstellung von Kohlensäureestern von Mono- oder Polyalkoholen, das dadurch gekennzeichnet ist, daß man Harnstoff mit Mono- oder mit Polyalkoholen im Molverhältnis von mindestens 1 zu 2, in Gegenwart von $10^{-3}$ bis 5 Mol %, bezogen auf Mole Harnstoff, eines Katalysators umsetzt, wobei man unter Rühren auf Temperaturen von mindestens 120 °C erwärmt, während 5 bis 10 Stdn. langsam auf 270 °C erwärmt und ausreagieren läßt.

Gegenüber der bekannten Umsetzung von sehr aktivierten Harnstoffen mit Alkoholen und Phenolen (siehe Staab, Angew. Chem. 68 (1956) Seite 754, Staab, Liebigs Ann. Chem. 609 (1957) Seite 75 ff und Seite 83 ff, K. Schlögl und H Woidich, Mh. Chem. 87 (1956) 679 sowie J. Derkosch, K. Schlögl und H. Woidich, Mh. Chem. 88 (1957) 35 und FR-PS 1 208 196) ist es überraschend, daß auch Harnstoff selbst sich mit Alkoholen gemäß dem erfindungsgemäßen Verfahren umsetzen läßt.

Aus der « Zeitschrift für Naturforschung, Band 1, 1946, Seiten 518 ff » ist die Umsetzung von Harnstoffen mit Alkoholen zu Urethanen bekannt. Eine Weiterreaktion der Urethane zu Kohlensäureestern ist dort nicht beschrieben. Mit den in dieser Literaturstelle angegebenen Katalysatoren lassen sich bei Verlängerung der Reaktionszeit geringe Mengen Carbonat durch Gaschromatographie nachweisen (s. « Angewandte Chemie », 92 Jahrgang 1980, Heft 9, Seiten 742-743, nachveröffentlicht).

In der US-Patentschrift 2 834 799 ist die Reaktion von Harnstoff mit Alkoholen und $BF_3$ zu Urethanen und weiter zu Carbonaten beschrieben, wobei hierfür pro Mol Harnstoff normalerweise 2 Mol $BF_3$ als Desaminierungsagenz notwendig sind (Spalte 1, Zeilen 32 bis 58), in besonderen Fällen zumindest 1 Mol Desaminierungsagenz pro Mol Harnstoff (s. Spalte 4, Zeile 6 und Spalte 4, Zeilen 38 bis 40). Bei Einsatz von Harnstoff-Bortrifluorid-Komplexen können pro Mol Bortrifluorid 5 Mol Urethane hergestellt werden (Spalte 3, Zeile 45 von US-PS 2 834 799).

Gegenüber dem Stand der Technik war das erfindungsgemäße Verfahren jedoch nicht naheliegend, das Elektronenakzeptoren oder Elektronendonatoren oder Kombinationen davon in Mengen von $10^{-3}$ bis 5 Mol-% pro Mol Harnstoff als Katalysatoren verwendet.

Als Monoalkohole bzw. Polyalkohole kommen vorzugsweise primäre Alkohole in Betracht, die eine beliebige Anzahl von C-Atomen enthalten können. Geeignete Alkohole sind aliphatische, cycloaliphatische, araliphatische und heterocyclische Alkohole. Polyalkohole im Sinne vorliegender Erfindung sind beispielsweise solche mit 2 bis 3 alkoholischen OH-Gruppen, wobei bei entsprechendem Abstand der alkoholischen OH-Gruppen im Molekül cyclische Kohlensäureester entstehen können.

Die geeigneten Monoalkohole haben vorzugsweise 1 bis 20 C-Atome.

Beispiele für Monoalkohole sind Methanol, Äthanol, Propanole, Butanole, Pentanole, Hexanole, Octanole, Stearylalkohol, Methylolcyclohexan, Benzylalkohol, 2-Phenyläthanol, 2-Naphthyläthanol, Furfurylalkohol etc.

Geeignete Polyalkohole haben vorzugsweise 5 bis 20 C-Atome.

Beispiele für Polyalkohole sind Hexan-1,6-diol, Decandiole, 1,4-Dimethylolcyclohexan, 1,4-Dimethylolbenzol, 3(4), 8(9) Bis (hydroxymethyl)-tricyclo-[5,2,1,$0^{2:6}$] decan

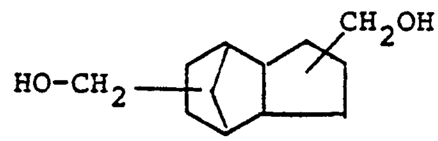

und Bis-hydroxymethyl-tetrahydrofurane etc.

Erfindungsgemäße geeignete Katalysatoren sind beispielsweise Verbindungen der 1. bis 6. Haupt- und Nebengruppen sowie der 7. und 8. Nebengruppen des Periodischen Systems der Elemente. (Siehe Hollemann-Wiberg, Lehrbuch der Anorganischen Chemie, 37-39, Auflage, Verlag Walter De Gruyter u. Co., Berlin 1956).

Erfindungsgemäß geeignete Verbindungen der 1. Haupt- und 1. Nebengruppe sind sowohl salzartige als auch covalente Verbindungen der Metalle dieser Gruppen des periodischen Systems wie LiBr, Butyllithium, LiCl, LiJ, $NaOCH_3$, CuCl, $AgOCO\text{-}CH_3$ etc.

Erfindungsgemäß geeignete Verbindungen der 2. Haupt- und 2. Nebengruppe sind sowohl salzartige als auch covalente Verbindungen der Metalle dieser Gruppen des periodischen Systems wie $MgCl_2$, $Be(O\text{-}CO\text{-}CH_3)_2$, Grignard-Verbindungen, $ZnCl_2$, $ZnSO_4$, $Mg(OC_2H_5)_2$ etc.

Erfindungsgemäß geeignete Verbindungen der 3. Haupt- und 3. Nebengruppe sind insbesondere covalente Verbindungen der Elemente dieser Gruppen des periodischen Systems wie B $(O{-}C_6H_5)_3$, $B(C_6H_5)_3$, $Al(OR)_3$, (R = aliphatischer oder aromatischer Kohlenwasserstoffrest) etc.

Erfindungsgemäß geeignete Verbindungen der 4. Haupt- und 4. Nebengruppe sind insbesondere covalente Verbindungen der Elemente dieser Gruppen des periodischen Systems wie Orthotitanate (z.B. Tetrabutyl-orthotitanat), Orthostannate (z.B. Tetraphenyl-orthostannat) etc.

Erfindungsgemäß geeignete Verbindungen der 5. Haupt- und 5. Nebengruppe sind insbesondere covalente Verbindungen der Elemente dieser Gruppen des periodischen Systems wie Amine (z.B. Diazabicyclooctan), Phosphine (z.B. Triphenylphosphin, Tri-n-octylphosphin), Phosphinoxide (z.B. Triphenylphosphinoxid) etc.

Erfindungsgemäß geeignete Verbindungen der 6. Haupt- und 6. Nebengruppe sind sowohl covalente als auch salzartige Verbindungen der Elemente dieser Gruppen des periodischen Systems wie Thioäther (z.B. Diphenylsulfid) Thiolate (z.B. Na-thiophenolat) etc.

Erfindungsgemäß geeignete Verbindungen der 7. Nebengruppe sind sowohl covalente als auch salzartige Verbindungen der Elemente dieser Gruppe des Periodischen Systems (z.B. Mangan (II) acetat).

Erfindungsgemäß geeignete Verbindungen der 8. Nebengruppe sind sowohl covalente als auch salzartige Verbindungen der Elemente dieser Gruppe des Periodischen Systems (z.B. Eisen (III) acetylacetonat).

Besonders bevorzugte Katalysatoren für das erfindungsgemäße Verfahren sind Kombinationen der vorstehend genannten Verbindungen, wobei Elektronendonator- und Elektronenakzeptor-Eigenschaften der Katalysatorkombination aufeinander abzustimmen sind, d.h. Kombinationen von Elektronendonatoren und Elektronenakzeptoren einzusetzen sind.

Beispiele derartiger Kombinationen sind Aluminium-alkoholate in Kombination mit Aminen, Phosphinen oder Phosphinoxiden, oder Magnesiumalkoholate in Kombination mit Thioäthern.

Die erfindungsgemäß geeigneten Katalysatoren werden in Mengen von $10^{-3}$ bis 5 Mol-%, bezogen auf Mole an jeweils eingesetzten Harnstoffen eingesetzt, und zwar gilt dies sowohl für den Einsatz von einzelnen Katalysatoren als auch von Katalysatorkombinationen. Das molare Verhältnis der Katalysatorarten in den Katalysatorkombinationen kann zwischen 1:10 und 10:1 variieren.

Zur Durchführung der erfindungsgemäßen Reaktion werden die Komponenten, d.h. Harnstoff, Alkohol und Katalysator zusammen unter Rühren auf Temperaturen von mindestens 120 °C erwärmt, während 5 bis 10 Stdn. langsam auf 270 °C erwärmt und ausreagieren lassen. Anschließend wird der Ansatz bei niedrigerer Temperatur im Vakuum schonend destillativ getrennt und der Kohlensäureester isoliert, wobei die Ausbeuten sehr gut sind.

Da im allgemeinen die Alkoholkomponente im Überschuß angewandt wird, variiert das Molverhältnis von Harnstoff zu Alkohol zwischen 1:2 und etwa 1:5, vorzugsweise zwischen 1:2 und 1:3.

Die nach dem erfindungsgemäßen Verfahren hergestellten Kohlensäureester sind bekannte Verbindungen und eignen sich bekanntlich als Lösungsmittel in der Organischen Chemie sowie als Zwischen- bzw. Ausgangsprodukte für die verschiedensten chemischen Reaktionen.

Insbesondere eignen sie sich in bekannter Weise (Vgl. DT-PS 1031512, Beispiel 1) zur Synthese von Polycarbonaten durch Umsetzung mit Diolen.

Die Herstellung von Oligo- und Polycarbonaten läßt sich einstufig nach dem erfindungsgemäßen Verfahren aus den genannten Harnstoffen, den genannten Monoalkoholen und primären Dialkoholen erreichen, wobei als Dialkohole aliphatische, cycloaliphatische und araliphatische geeignet sind unter dem Vorbehalt, daß die Bildung cyclischer Carbonate unter den Verfahrensbedingungen praktisch nicht auftritt.

Gegenstand der vorliegenden Erfindung ist somit auch ein Verfahren zur Herstellung von Oligo- und von Polycarbonaten, das dadurch gekennzeichnet ist, daß man Harnstoff mit primären Dialkoholen im Molverhältnis von etwa 1,5:1 bis etwa 1:1,5 und mit Monoalkoholen in Gegenwart von s.MS $10^{-3}$ bis 5 Mol %, bezogen auf Mole Harnstoff, von Katalysatoren bei Reaktionstemperaturen zwischen 120 °C und 270 °C umsetzt, unter dem Vorbehalt, daß die Bildung cyclischer Carbonate unter den Verfahrensbedingungen praktisch nicht auftritt.

Vorzugsweise ist hierbei das Molverhältnis von Harnstoff zu Dialkoholen etwa 1:1; die Molmenge an Monoalkoholen bezogen auf Molmenge an Dialkoholen, richtet sich nach der jeweils angestrebten Kettenlänge und kann, da im Überschuß eingesetzt, destillativ entfernt werden. Durch die Wahl der Reaktionstemperatur und der Reaktionszeit wird das Molekulargewicht der Oligo- bzw. Polycarbonate außerdem in bekannter Weise reguliert. Nach dem erfindungsgemäßen Verfahren sind somit auf einfache Weise Oligo- und Polycarbonate von primären Dialkoholen mit verschiedenen Molekulargewichten herstellbar.

Für die, für die erfindungsgemäße Oligo- und Polycarbonatherstellung geeigneten, Monoalkohole und Katalysatoren sind die eingangs gemachten Definitionen zur Erläuterung der erfindungsgemäßen Herstellung von Kohlensäureestern ebenfalls zutreffend.

Für die erfindungsgemäße Oligo- und Polycarbonatherstellung sind je nach Ansatz und Temperaturführung zwischen 2 und 10 Stunden erforderlich. Das jeweils gewünschte Ende der Reaktion kann beispielsweise viskosimetrisch ermittelt werden.

Als primäre Dialkohole sind vorzugsweise solche mit 5 bis 20 C-Atomen geeignet; Beispiele sind die bereits eingangs im Zusammenhang mit der erfindungsgemäßen Herstellung von Kohlensäureestern genannten, wie Hexan-1,6-diol oder 1,4-Dimethylol-cyclohexan.

Die übrigen Reaktionsbedingungen der Oligo bzw. Polycarbonatherstellung, also Einsatz und Menge der einzusetzenden Katalysatoren sowie die Temperaturführung entsprechen denen der bereits beschriebenen Herstellung der monomeren Kohlensäureester.

Die nach dem erfindungsgemäßen Verfahren erhaltenen Oligo- und Polycarbonate sind im Prinzip bekannt und geeignet in bekannter Weise, zur Herstellung von Formkörpern und Folien sowie als Beschichtungen und Additive für andere Kunststoffe. Die erfindungsgemäß erhältlichen Oligo- und Polycarbonate sind somit geeignet für die bekannten Einsatzgebiete der thermoplastischen Polycarbonate, also etwa bezüglich der Oligocarbonate als sekundäre Weichmacher für beispielsweise PVC sowie etwa zur Modifizierung von hochmolekularen

thermoplastischen Polycarbonaten beispielsweise gemäß US-PS 3 166 606.

Beispiel 1

Darstellung eines niedermolekularen Kohlensäureesters

30,95 g Harnstoff, 185,0 g 2-Ethylhexanol-(1), 0,69 g Triphenylphosphin und 2,6 ml einer 20 %igen Lösung von Diisobutylaluminiumhybrid in Toluol wurden zusammen erwärmt. Bei ca. 150 °C begann die weißlich-trübe Mischung zu sieden. Während 1,5 h wurde die Temperatur auf 192 °C erhöht; es waren zu diesem Zeitpunkt 10,46 g $NH_3$ abgeschieden. Während weiteren 7 h wurde die Temperatur bis auf 269 °C erhöht; es waren dann 16,23 g $NH_3$ abgeschieden und 56,02 g Flüssigkeit abdestilliert, die bis auf wenig gelöstes Urethan, Harnstoff und $NH_3$ aus 2-Ethylhexanol-1 bestand. Anschließend wurde die Mischung fraktioniert; es resultierten

eine Fraktion von 20,67 mit $Kp_{14}$ 81,5 (Alkohol) und eine Fraktion von 128,80 mit ($Kp_{\sim 0,05}$ 98—102 Bis-(2-ethylhexyl)-carbonat, Ausbeute 87,9 %, bezogen auf eingesetzten Harnstoff.

| | | ber. | gef. |
|---|---|---|---|
| Analyse | % C | 71,28 | 72,59 |
| | % H | 11,96 | 11,92 |
| | % O | 16,76 | 16,54 |

Beispiel 2

Darstellung eines Polycarbonates

15 g Harnstoff, 30,26 g 1,4-Bis-(hydroxymethyl)-cyclo-hexan, 34 g Isoonylalkohol, 0,34 g Triphenylphosphin und 1,2 ml einer 20 %igen Lösung von Diisobutylaluminiumhybrid in Toluol wurden zusammengeschmolzen und erwärmt. Bei ca. 170 °C war die Schmelze fast klar und begann lebhaft zu sieden. Während 45 Min. wurde die Temperatur auf 225 °C gesteigert; zu diesem Zeitpunkt waren 4,92 g $NH_3$ abgeschieden. Dann wurde die Temperatur während 2,5 h kontinuierlich bis auf 260 °C erhöht; es waren dann 7,37 h $NH_3$ abgeschieden.

Nach Abkühlen der Schmelze auf 150 °C wurde Wasserstrahlvakuum angelegt und während 30 Min. die Temperatur auf 260 °C gesteigert; hierbei destillierte der Isononylalkohol ab und die Schmelze wurde viskos. Danach wurde die Reaktionsmischung für 3 h im Ölpumpenvakuum auf 250 °C erwärmt, wobei der Rest Isononylalkohol, etwas Diol und Diisononylcarbonat abdestillierten. Es blieben 32,78 g klares, nichtkristallisierendes Polycarbonat zurück ($M_n \sim 11\,000$).

Die Mn-Bestimmung erfolgte durch Dampfdruckosmometrie. Das Polycarbonat hat ein $\eta_{rel}$ (gemessen als 0,5 %ige Lösung in Methylenchlorid bei 25 °C) von 1,26.

Beispiel 3

10 g Harnstoff, 70 g Isononanol, 0,5 ml Tetrabutylorthotitanat und 0,2 g LiBr wurden analog Beispiel 1 umgesetzt. Ausbeute an Diisononylcarbonat 57 %.

Beispiel 4

10 g Harnstoff, 70 g 2-Ethylhexanol-(1), 0,57 Magnesium-acetat und 1,03 g Triphenylphosphin wurden analog Beispiel 1 umgesetzt. Die Ausbeute an Bis-(2-ethylhexyl)-carbonat betrug 41 %.

Beispiel 5

10 g Harnstoff, 70 g 2-Ethylhexanol-(1), 1,01 g Eisen (III)-acetylacetonat und 0,71 g Triphenylphosphin wurden analog Beispiel 1 umgesetzt. Die Ausbeute an Kohlensäureester betrug 76 %.

Beispiel 6

10 g Harnstoff, 70 g 2-Ethylhexanol-(1), 0,5 g Chrom (III)-acetat und 1,0 g Triphenylphosphin wurden analog Beispiel 1 umgesetzt. Die Ausbeute an Kohlensäureester betrug 43 %.

**Ansprüche**

1. Verfahren zur Herstellung von Kohlensäureestern von Mono- oder Polyalkoholen, dadurch gekennzeichnet daß man Harnstoff mit Mono- oder mit Polyalkoholen im Molverhältnis von mindestens 1 zu 2 in Gegenwart von $10^{-3}$ bis 5 Mol %, bezogen auf Mole Harnstoff, eines Katalysators umsetzt, wobei man unter Rühren auf Temperaturen von mindestens 120 °C erwärmt, während 5 bis 10 Stdn. langsam auf 270 °C erwärmt und ausreagieren läßt.

2. Verfahren zur Herstellung von Oligo- und von Polycarbonaten, dadurch gekennzeichnet, daß man Harnstoff mit primären Dialkoholen im Molverhältnis von etwa 1,5: 1 bis etwa 1: 1,5 und mit Monoalkoholen in Gegenwart von $10^{-3}$ bis 5 Mol %, bezogen auf Mole Harnstoff, von Katalysatoren bei Reaktionstemperaturen zwischen 120 °C und 270 °C umsetzt, unter dem Vorbehalt, daß die Bildung cyclischer Carbonate unter den Verfahrensbedingungen praktisch nicht auftritt.

3. Verfahren gemäß Anspruch 2, dadurch gekennzeichnet, daß das Molverhältnis von Harnstoff zu Dialkohol etwa 1: 1 ist.

**Claims**

1. Process for the preparation of carbonic acid esters of monoalcohols or polyalcohols, characterised in that urea is reacted with monoalcohols or with polyalcohols in the molar ratio of at least 1: 2 in the presence of $10^{-3}$ to 5 mol %, relative to the number of mols of urea, of a catalyst, in which reaction the components are warmed, whilst stirring, to temperatures of at least 120 °C, then warmed slowly to 270 °C for 5 to 10 hours and allowed to finish reacting.

2. Process for the preparation of oligocarbo-

nates and polycarbonates, characterised in that urea is reacted with primary dialcohols in the molar ratio of about 1.5 : 1 to about 1: 1.5 and with monoalcohols in the presence of $10^{-3}$ to 5 mol %, relative to the number of mols of urea, of catalysts, at reaction temperatures between 120 °C and 270 °C, with the proviso that virtually no formation of cyclic carbonates occurs under the process conditions.

3. Process according to Claim 2, characterised in that the molar ratio of urea to dialcohol is about 1: 1.

**Revendications**

1. Procédé de préparation d'esters d'acide carbonique de mono- ou poly-alcools, caractérisé en ce que l'on fait réagir de l'urée avec des mono- ou des poly-alcools dans un rapport molaire d'au moins 1 à 2 en présence de $10^{-3}$ à 5 moles %, par rapport aux moles d'urée, d'un catalyseur, en chauffant sous agitation à des températures d'au moins 120 °C, en portant lentement en 5 à 10 heures à 270 °C et en laissant réagir complètement à cette température.

2. Procédé de préparation d'oligo- et poly-carbonates, caractérisé en ce que l'on fait réagir de l'urée avec des dialcools primaires dans un rapport molaire d'environ 1,5: 1 à 1: 1,5 et avec des mono-alcools en présence de $10^{-3}$ à 5 moles %, par rapport aux moles d'urée, de catalyseurs, à des températures de réaction de 120 à 270 °C, sous réserve qu'il ne se forme pratiquement pas de carbonates cycliques dans les conditions du procédé.

3. Procédé selon la revendication 2, caractérisé en ce que le rapport molaire de l'urée au dialcool est d'environ 1: 1.